# EUROPEAN PATENT APPLICATION

(11) **EP 4 194 022 A1**
(43) Date of publication of application: **14.06.2023**
(21) Application number: 21213491.0
(22) Date of filing: 09.12.2021
(51) Int. Cl.: A61M 1/06

(54) **A BREAST PUMP**

(71) Applicant: Koninklijke Philips N.V., 5656 AG Eindhoven (NL)
(72) Inventor: DOBRUSSKIN, Christoph, Eindhoven (NL); BOURQUIN, Yannyk Parulian Julian, Eindhoven (NL); WIJNOLTZ, Anna Louise, Eindhoven (NL)
(74) Representative: Philips Intellectual Property & Standards

(57) **Abstract**

A breast pump comprises a cover layer for fitting over a portion of a breast thereby to create a cavity. The breast pump is arranged such that the cavity is at least partially filled with expressed milk before the expressed milk flows to the outlet. An actuator is used to apply vibrations to the breast through the expressed milk.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

Recently, there is a trend towards wearable breast pumps. These devices are generally in a smaller form factor and can be fully worn on the user's body, allowing the user to move around freely. Some of these are small enough to fit into a user's bra. A motor, battery and milk container are for example integrated to form a single unit.

It is also known to apply a vibration to the breast during use of a breast pump, to stimulate milk expression. It is reported that the use of vibrations can increase the milk expression efficiency.

However, it is difficult to create vibrations using the vacuum pump because the vibrations are damped by the volume of air between the pump and the breast. Thus, it is difficult to achieve the desired transfer of vibrations to the breast without complicating the design of the breast pump.

### SUMMARY OF THE INVENTION

The invention is defined by the claims.

According to examples in accordance with an aspect of the invention, there is provided a breast pump, comprising:
a cover layer for fitting over a portion of a breast thereby to create a cavity (46) over said portion;
an outlet from the cavity, wherein the breast pump is arranged such that the cavity is at least partially filled with expressed milk before the expressed milk flows to the outlet;
an actuator; and
a controller for controlling the actuator,
wherein the controller is adapted to control the actuator to apply, during a vibration mode of the breast pump, vibrations to the breast at least partially through the expressed milk.

The actuator for example force or pressure pulses to the cover layer, or from the cover layer to the internal volume of the cavity.

This breast pump provides a liquid coupling between a cover layer and the breast, via the milk collected within the cavity formed by the cover layer. In this way, vibrations can be coupled more effectively to the breast tissue to provide stimulation of the breast based on the transfer of vibration.

The portion of the breast includes the nipple so that expressed milk enters the cavity. The outlet from the cavity is for example designed so that the cavity at least partially fills with expressed milk before expressed milk flows to the outlet. However, other arrangements may be used to ensure milk is retained in the cavity, such as a valve or pump arrangement.

Force or pressure may be applied to the outside of the cavity (i.e. to the cover layer) via an actuation liquid or an actuation gas to which pressure is applied, or a mechanical force may be applied directly to the cavity for example i.e. via the cover layer. In all cases, the vibratory pulses are sent through the cavity, and hence through the expressed milk within the cavity, to the user. This hydraulic action will have a stronger effect on the skin of the user (at the portion of the breast) than a pneumatic action. Thus, pulses are applied to the breast through the expressed milk.

In one set of examples, the cover layer comprises a diaphragm, and the breast pump further comprises:
a cover for fitting over the diaphragm, wherein an actuation volume is defined between the diaphragm and the cover;
a pressure source for applying a pressure to the actuation volume; and
wherein the controller is adapted to control the actuator to apply, during the vibration mode of the breast pump, pressure pulses to the actuation volume.

The cover is sealed over the diaphragm, and an actuation volume is defined between them. Thus, pressure may be transferred to the diaphragm via the actuation volume. The actuation volume may contain a gas (e.g. air) or a liquid.

The controller is for example adapted to control the pressure source to apply, during a milk expression mode, a cyclic pressure waveform to the actuation volume.

Thus, there is a normal expression mode with a cyclic pressure waveform, as well as a vibration mode.

The controller is for example adapted to control the actuator to apply the pulses of the vibration mode before the cyclic pressure waveform of the expression mode. Thus, the vibration mode may take place before the expression mode. Of course in that case, the vibration pulses will only be transferred through the expressed milk once milk has started to flow.

The controller may then additionally apply the pulses of the vibration mode simultaneously with the cyclic pressure waveform of the expression mode. Thus, the stimulation achieved by the vibration mode may be used in combination with the cyclic waveform during expression, to improve the efficiency of the milk expression.

The pulses for example have a frequency in the range 1 to 1kHz, for example 10 to 250Hz, and the cyclic repetition frequency is below 2Hz. For example, the pulses typically have a higher frequency than the cyclic repetition frequency of the cyclic pressure waveform.

In one example, the actuator is the pressure source, and the controller is adapted to control the pressure source to apply the pulses to the actuation volume. Thus, the pressure source may be controlled in different ways to implement different modes of operation (in one mode it functions as the actuator and in another mode it functions as the pressure source for a cyclic expression waveform), and multiple pressure source control signals may be superposed to implement a combination of modes.

In another example, the breast pump comprises a coupling tube between the pressure source and the cover. Instead of modulating the output of the pressure source to implement the vibrations, the actuator of the breast pump comprises a vibrator for imparting the pulses to the actuation volume via the coupling tube.

In one example, the vibrator is for deforming the coupling tube. It may for example press against the coupling tube to create a pressure pulse.

In another example, the vibrator is for applying pulses to the fluid in a branch which connects to the coupling tube.

Instead of coupling vibrations to a coupling tube from the pressure source or to a branch leading to the coupling tube, a vibrator may be used for imparting the pulses to the actuation volume, from the outside of the cover.

By creating a closed hydraulic system, vibrations may be generated and applied to any part of that closed system.

The vibrator may for example comprise a valve. The actuation of the valve will create internal pressure pulses.

The breast pump for example further comprises a milk collection container and a valve between the outlet from the cavity and the milk collection container. The valve is for example a flap valve such as a duckbill valve. It may be formed as an integrated part of the diaphragm.

The breast pump for example comprises a wearable breast pump.

The invention also provides a method of operating a breast pump for the non-therapeutic expression of milk, wherein the breast pump comprises:
a cover layer for fitting over a portion of a breast thereby to create a cavity over said portion;
an outlet from the cavity, wherein the breast pump is arranged such that the cavity is at least partially filled with expressed milk before the expressed milk flows to the outlet;
an actuator; and
a controller for controlling the actuator,
wherein the method comprises operating the controller such that the actuator applies, during a vibration mode of the breast pump, vibrations to the breast at least partially through the expressed milk.

The cover layer for example comprises a diaphragm, and the breast pump comprises a cover for fitting over the diaphragm, with an actuation volume is defined between the diaphragm and the cover, and a pressure source for applying a pressure to the actuation volume, wherein the method comprises operating the controller such that the pressure source applies, during a milk expression mode, a cyclic pressure waveform to the actuation volume.

The invention also provides a computer program comprising computer program code means which is adapted, when said program is run on processor of the breast pump of defined above, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows a first example of a breast pump to which the invention may be applied;
Figure 4 shows a second example of a breast pump to which the invention may be applied;
Figure 5 shows a diaphragm design which may be used to implement another breast pump design to which the invention may be applied; and
Figure 6 shows a third example of a breast pump to which the invention may be applied.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump which comprises a cover layer for fitting over a portion of a breast thereby to create a cavity. The breast pump is arranged such that the cavity is at least partially filled with expressed milk before the expressed milk flows to the outlet. An actuator is used to apply vibrations to the breast through the expressed milk.

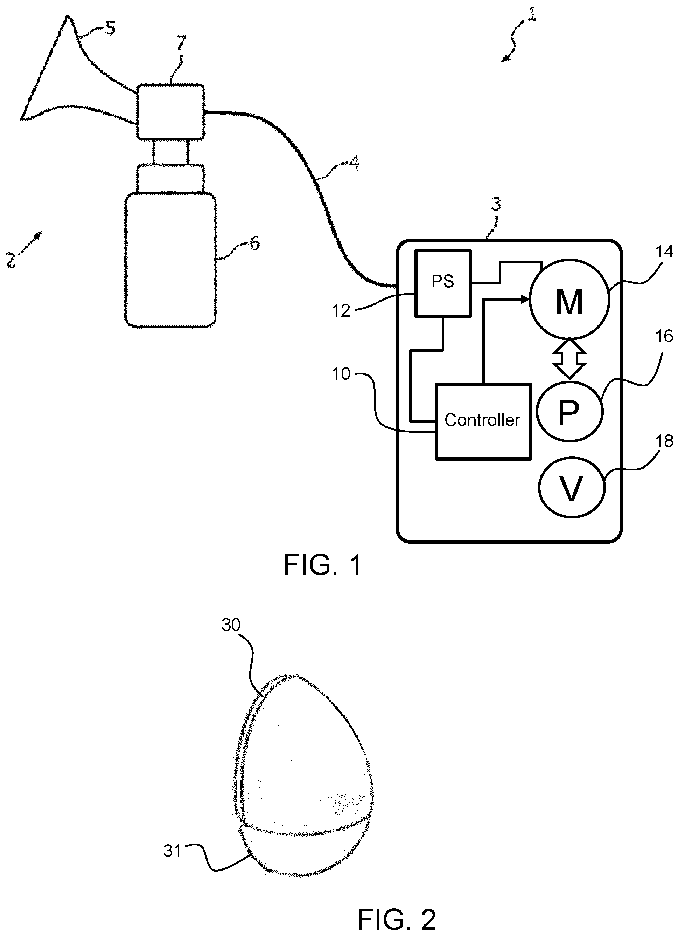

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection container 31.

The invention provides a simpler system for stimulating the milk ejection reflex and performing collection of expressed milk. In particular, the invention combines a contact stimulation unit to stimulate the milk ejection reflex with a more simple negative pressure source which applies a static negative pressure.

The invention is of particular interest for a wearable breast pump, but it may be applied to other types of breast pump as outlined above to improve ease of use.

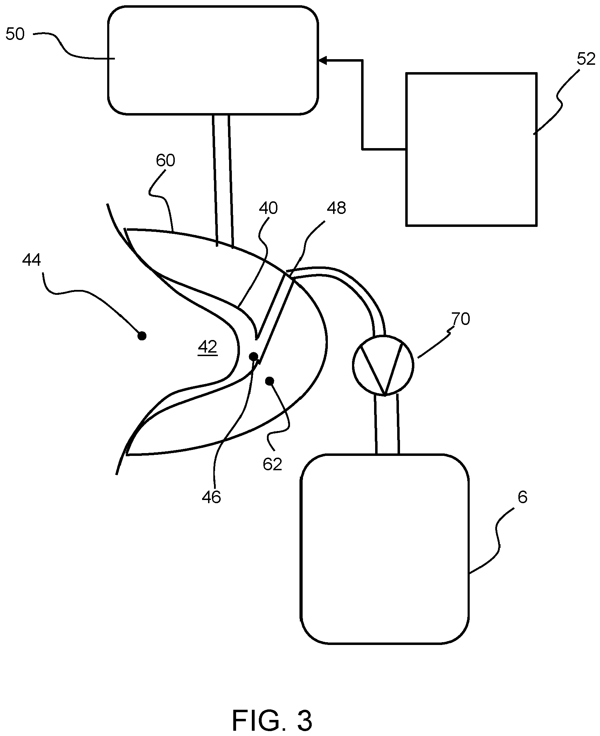

Figure 3 shows a first example of a breast pump.

In this particular example, the breast pump comprises a diaphragm 40 for fitting over a portion of the breast, including at least the nipple 42 of the breast 44, thereby to create a cavity 46 over at least the nipple. The diaphragm is sealed over the breast to define a closed and sealed cavity. The diaphragm is used to transfer a pressure waveform to the breast in known manner, for implementing an expression mode, and optionally also a separate stimulation mode, as discussed above.

More generally, the diaphragm is one possible example of a cover layer, for forming a cavity over a portion of the breast. The cavity may instead be separate to the arrangement by which an expression waveform is applied to the breast. However, the cavity formed by such a cover layer is in all cases able to receive expressed milk. In particular, the breast pump is arranged such that the cavity is at least partially filled with expressed milk before the expressed milk flows to the milk collection container 6.

This filling of the cavity may be achieved by means of a valve or pump arrangement. However, in the example shown, it is the design of the outlet 48 from the cavity that provides that the cavity 46 is at least partially filled with expressed milk before the expressed milk flows to the outlet 48. Preferably, the cavity is fully filled with milk.

This can be achieved by providing the cavity outlet 48 above the top of the cavity 46 as shown so that air is displaced and expelled from the cavity by the expressed milk before the expressed milk reaches the outlet 48.

In the example shown, the outlet is at the front (i.e. the nipple end) of the cavity. However the outlet may be at the opposite, back, end of the cavity, so that milk flow from the front to the back, thereby filling the cavity before reaching the outlet.

An actuator 50 is provided for delivering vibrations to the breast. In the example shown, this is achieved by applying a force or pressure to the cavity, in particular via the cover layer (i.e. the diaphragm in this particular design). Alternatively, the actuator may be part of the cover layer, and may this apply force or pressure directly to the cavity volume. A controller 52 is provided for controlling the actuator.

In the example shown. the controller is adapted to control the actuator 50 to apply, during a vibration mode of the breast pump, force or pressure pulses to the diaphragm 40. There is a liquid coupling between the diaphragm 40 and the breast 44, via the milk collected within the cavity 46. As a result, the vibrations (the force or pressure pulses) can be coupled more effectively to the breast tissue to implement a stimulation mode based on the transfer of vibration. In particular, a hydraulic transfer will have a stronger effect on the skin of the user (at the nipple and optionally part of the breast) than a pneumatic action, with reduced loss.

The actuator 50 may apply mechanical force or pressure pulses directly to the diaphragm, by using a vibrating system in contact with the diaphragm.

Figure 3 instead shows an implementation in which the force or pressure pulses are applied to the diaphragm through an intermediate chamber, typically containing air, but which could instead contain an actuation liquid.

For this purpose, the breast pump comprises a cover 60 which seals over the diaphragm, and an actuation volume 62 is defined between the diaphragm and the cover. Thus, pressure may be transferred to the diaphragm 40 via the actuation volume. The actuation volume may contain a gas (e.g. air) or a liquid.

There is then a pressure source for applying a pressure to the actuation volume 62. In the example of Figure 3, the pressure source is implemented by the actuator 50. Thus, However, in other examples, the pressure source and the actuator 50 may be separate components.

The actuator 50 thus applies pressure pulses to the actuation volume to implement the vibration mode of the breast pump.

In preferred examples, the pressure source is also controlled to apply, during a milk expression mode, a cyclic pressure waveform to the actuation volume. Thus, there is a normal expression mode with a cyclic pressure waveform, as well as a vibration mode.

The vibration mode is for example applied before the cyclic expression mode, and is used to stimulate the milk ejection reflex. Until milk is expressed, there will be no milk in the cavity and hence the vibrations are not yet transferred through the expressed milk. The vibrations of the vibration mode continue, combined with the cyclic pressure waveform of the expression mode. Thus, the vibration stimulation is used in combination with the cyclic waveform during expression, to improve the efficiency of the milk expression.

The vibration pulses for example have a frequency in the range 1 to 1kHz, for example 10 to 250Hz. As mentioned above, the cyclic repetition frequency is typically below 2Hz.

The vibration pulses typically have a higher frequency than the cyclic repetition frequency of the cyclic pressure waveform.

When the actuator is the pressure source as shown in Figure 3, the pressure source may thereby be controlled in different ways to implement different modes of operation. In one mode it functions as the actuator, and in the other mode it functions as the pressure source for a cyclic expression waveform.

Figure 3 shows the milk collection container 6 and a valve 70 between the outlet 48 of the cavity 46 and the milk collection container 6. The valve 70 is for example a flap valve such as a duckbill valve. It may be formed as an integrated part of the diaphragm.

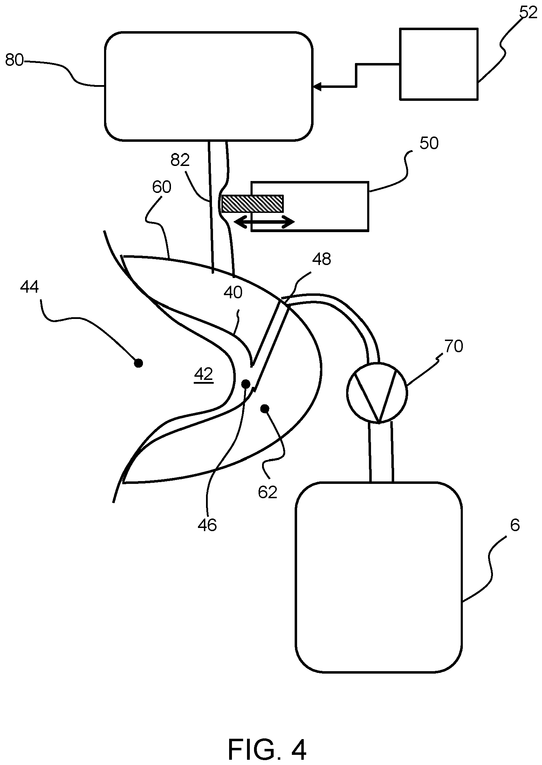

Figure 4 shows a second example in which the actuator 50 comprises a vibrator which is separate to the pressure source 80. In this example, the breast pump comprises a coupling tube 82 between the pressure source 80 and the cover 60. Instead of modulating the output of the pressure source to implement the vibrations, the actuator 50 of the breast pump comprises a vibrator for imparting the pulses to the actuation volume via a deformation of the coupling tube 82.

This for example enables better control of the vibration.

The vibration generated may be controlled in terms of timing (e.g. only during stimulation phase or during specific phase of a pumping cycle) and in terms of amplitude. It may also be activated when the pump is off e.g. before a pumping session.

The vibrator for example comprise a mechanical drive system for deforming the coupling tube. It may for example comprise a piston which presses against the coupling tube to create a pressure pulse which is transferred to the fluid within the tube 82, as schematically shown in Figure 4.

A mechanical vibrator may be a motor with an eccentric load, a motor with sprung rollers, a Lorentz actuator, a microphone, a magnetic actuator, or a piezoelectric actuator.

In an alternative example, the vibration is for applying pressure pulses to fluid in a leg of a T-branch or Y-branch which connects to the coupling tube 82. The vibrator may thus apply pressure pulses to the branch, which then couple to the coupling tube. In this way, separate pressure sources are provided for the vibration mode and for the normal cyclic pressure mode for expression. The vibrator is in that case a pump.

In yet another example, instead of coupling vibrations to the coupling tube or to a branch to the coupling tube, a vibrator may be used for imparting mechanical vibrations to the actuation volume 62, from the outside of the cover 60. In a further alternative example, a vibrator may be placed at the milk outlet leading to the milk container.

Thus, it will be understood that by creating a closed hydraulic system, vibrations may be generated and applied to any part of that closed system that allows a hydraulic coupling via the expressed milk between the vibration generating element and the nipple or breast.

The vibrator may for example comprise a valve. The actuation of the valve will create internal pressure pulses. The outlet valve 70 may for example be used as the actuator, or there may be a valve at another location within the overall hydraulic system.

When the actuator is implemented as a pump (whether the same pump for creating a cyclic pressure waveform or a separate pump), it may comprise a vacuum pump or a hydraulic pump. A hydraulic pump may comprise a gear pump, a peristaltic pump, a rotary vane pump or a screw pump. These pump designs inherently generate micro oscillations.

The use of a hydraulic pump, and with a liquid in the activation volume, would result in a fully hydraulic system where the vibration generated at the pump or from the vibrator would be most optimally transferred to the hydraulic interface and breast.

The diaphragm may have various designs.

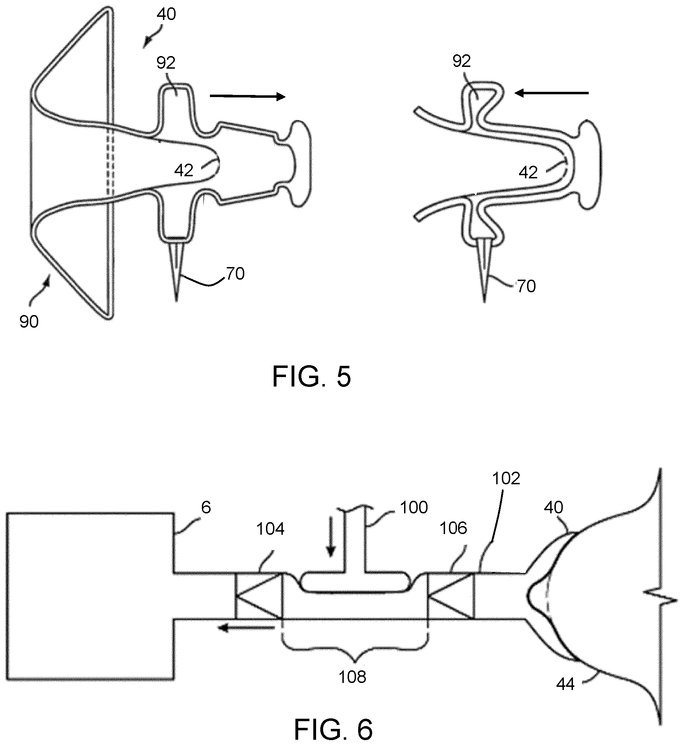

Figure 5 shows one possible diaphragm design which comprises a breast shield portion 90 which is funnel-shaped and which leads to a nipple tunnel 92 which is formed as an extensible chamber. When extending the chamber, a vacuum is generated. During milk expression the chamber is filled with fluid making it a hydraulic system.

Thus, in this case, the cavity has an adjustable volume to create an under pressure by physically adjusting the shape of the diaphragm, instead of using an external pressure source. The left image shows an extended chamber and the right image shows a compressed chamber. Thus, in this case, the force or pressure pulses may be applied to the diaphragm by controlling the shape of the diaphragm, for example using a motor rather than a pump.

Figure 6 shows another example of a breast pump design which may be adapted using the invention. This design is described in more detail in US 10688229.

Figure 6 shows a breast pump with a compression element 100 for compressing an outlet tube 102. Valves 104, 106 are at each side of the compression element 100 and hence adjacent opposite ends of a compression region 108 of the tube 102 where the tube is compressed by compression element 100. The diaphragm 40 is sealed to the breast 44, and pumping of expressed milk is performed by compressing region 108 with compression element 100. As the pressure increases due to the compression of region 108, milk is driven through one-way valve 104 as indicated by the leftward directed arrow. At the same time, one-way valve 106 prevents backward flow of the milk and maintains vacuum against the breast 44.

Next, the compression element is retracted away from the tube 102 and the tube 102 resiliently expands to increase vacuum (and hence drop the pressure). This closes the one-way valve 104 and opens the one-way valve 106 to extract milk from the breast and into the region 108. Extraction and pumping of milk can be continued by cycling between these two phases.

The milk container 6 in this design is preferably a flexible bag which starts in a collapsed state so that it does not take up any significant volume until milk is received therein.

During normal operation, as the pump mechanism (the compression element 100) operates and the tube 102 becomes mostly filled with milk, the breast pump system approaches a fully hydraulic system A vibration can then be applied to the breast as described above. The vibrations may be hydraulically introduced by modulating the drive of the compression element 100, or by modulating the valve 106 or by introducing vibration using an additional vibrating element.

In all examples above, the shape of the vibrations, including frequencies, amplitude and/or strength of the vibrations could be arranged to be adjustable, for example by user interface controls, to allow the user to adjust these parameters to his or her optimal comfort.

Alternatively, these shape parameters could be arranged to be adjusted automatically based on sensor input, for example the damping of the vibrations by the breast tissue could be measured and used to adjust the amplitude and/or strength. Furthermore, the shape parameters could be arranged to be adjusted based on a learning algorithm that uses milk output parameters such as speed of expression and amount of milk expressed collected over different sessions, with different vibration shape parameters to identify an optimal shape for a specific user.

Furthermore, an optimal frequency shape could be determined based by a learning system based on preferences shown by various users or optimized milk expression efficiency experienced by various users.

The various different diaphragm designs may each be considered to comprise a cover layer.

The breast pump for example comprises a wearable breast pump.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump, comprising:
a cover layer (40) for fitting over a portion of a breast thereby to create a cavity (46) over said portion;
an outlet (48) from the cavity, wherein the breast pump is arranged such that the cavity (46) is at least partially filled with expressed milk before the expressed milk flows to the outlet;
an actuator (50); and
a controller (52) for controlling the actuator,
wherein the controller (52) is adapted to control the actuator to apply, during a vibration mode of the breast pump, vibrations to the breast at least partially through the expressed milk.

2. The breast pump of claim 1, wherein the cover layer comprises a diaphragm, and the breast pump further comprises:
a cover (60) for fitting over the diaphragm, wherein an actuation volume (62) is defined between the diaphragm (40) and the cover (60);
a pressure source (50; 80) for applying a pressure to the actuation volume (62); and
wherein the controller is adapted to control the actuator (50) to apply, during the vibration mode of the breast pump, pressure pulses to the actuation volume (62).

3. The breast pump of claim 2, wherein the controller is adapted to control the pressure source (50; 80) to apply, during a milk expression mode, a cyclic pressure waveform to the actuation volume.

4. The breast pump of claim 3, wherein the controller is adapted to control the actuator to apply the pulses of the vibration mode:
before the cyclic pressure waveform of the expression mode; and/or
simultaneously with the cyclic pressure waveform of the expression mode.

5. The breast pump of claim 3 or 4, wherein the pulses have a frequency in the range 1 to 250Hz, for example 10 to 250Hz, and wherein the cyclic repetition frequency is below 2Hz, and preferably wherein the pulses have a higher frequency than the cyclic repetition frequency of the cyclic pressure waveform.

6. The breast pump of any one of claims 2 to 5, wherein the actuator (50) is the pressure source, and the controller is adapted to control the pressure source to apply the pulses to the actuation volume.

7. The breast pump of any one of claims 2 to 5, comprising a coupling tube (82) between the pressure source and the cover, wherein the actuator (50) comprises a vibrator for imparting the pulses to the actuation volume via the coupling tube (82).

8. The breast pump of claim 7, wherein the vibrator is for:
deforming the coupling tube (82); or
applying pulses to the fluid in a branch which connects to the coupling tube (82).

9. The breast pump of any one of claims 2 to 5, wherein the actuator comprises a vibrator for imparting the pulses to the actuation volume, from the outside of the cover (60).

10. The breast pump of any one of claims 7 to 9, wherein the vibrator comprises a valve.

11. The breast pump of any one of claims 1 to 10, wherein the breast pump further comprises a milk collection container (6) and a valve (70) between the outlet from the cavity and the milk collection container.

12. The breast pump of any one of claims 1 to 11, comprising a wearable breast pump.

13. A method of operating a breast pump for the non-therapeutic expression of milk, wherein the breast pump comprises:
a cover layer (40) for fitting over a portion of a breast thereby to create a cavity (46) over said portion;
an outlet (48) from the cavity, wherein the breast pump is arranged such that the cavity (46) is at least partially filled with expressed milk before the expressed milk flows to the outlet;
an actuator (50); and
a controller (52) for controlling the actuator,
wherein the method comprises operating the controller such that the actuator applies, during a vibration mode of the breast pump, vibrations to the breast at least partially through the expressed milk.

14. The method of claim 13, wherein the cover layer comprises a diaphragm, and the breast pump comprises:
a cover for fitting over the diaphragm, wherein an actuation volume is defined between the diaphragm and the cover; and
a pressure source for applying a pressure to the actuation volume,
wherein the method comprises operating the controller such that the pressure source to applies, during a milk expression mode, a cyclic pressure waveform to the actuation volume.

15. A computer program comprising computer program code means which is adapted, when said program is run on processor of the breast pump of any one of claims 1 to 12, to implement the method of claim 13 or 14.
